# EUROPEAN PATENT APPLICATION

(11) **EP 2 502 549 A1**
(43) Date of publication of application: **26.09.2012**
(21) Application number: 12158989.9
(22) Date of filing: 12.03.2012
(51) Int. Cl.: A61B 1/00, A61B 1/06, A61B 1/07, G02B 23/24, G02B 23/26, G02B 6/24

(54) **Endoscope apparatus**

(30) Priority: 25.03.2011 JP 2011067554
(71) Applicant: Fujifilm Corporation, Minato-ku Tokyo 106-8620 (JP)
(72) Inventor: Yoshida, Koji, Kanagawa, 258-8538 (JP); Kasamatsu, Tadashi, Kanagawa, 258-8538 (JP); Iwasaka, Masayuki, Kanagawa, 258-8538 (JP)
(74) Representative: Klunker . Schmitt-Nilson . Hirsch

(57) **Abstract**

To prevent excitation light output from an output end of a light guiding portion causing damage to the eye when the light guiding portion which guides the special light output from a light source is detached from the insertion portion. Provided is an endoscope apparatus including: an insertion portion that is inserted into a body so as to irradiate special light to a subject observation portion inside the body and receives light output from the subject observation portion by the irradiation of the special light; and a light guiding portion that is optically connected to the insertion portion and guides the special light output from a light source to the insertion portion, wherein an output end for the special light in the light guiding portion is equipped with a diffusing portion that diffuses the special light guided by the light guiding portion.

## Description

### BACKGROUND OF THE INVENTION

**1. Field of the Invention**

The present invention relates to an endoscope apparatus equipped with an insertion portion that irradiates a special light to a subject observation portion inside a body and receives light output from the subject observation portion by the irradiation of the special light.

**2. Description of the Related Art**

Hitherto, an endoscope system which observes tissue inside a body cavity has been widely known, and an electronic endoscope system which captures an ordinary image of a subject observation portion inside a body cavity by the irradiation of white light, acquires an ordinary image, and displays the ordinary image on a monitor screen has been widely put to practical use.

Then, as one example of these types of endoscope systems, an endoscope system is proposed which inputs ICG (indocyanine green) into a subject observation portion in advance and then irradiates excitation light of near-infrared light to the subject observation portion to acquire a fluorescence image of ICG for the purpose of observing blood vessels and blood flow under fat, lymph vessels, lymph flow, biliary tracts, bile flow and the like which do not show up on an ordinary image.

As an example of an endoscope system that acquires a fluorescence image, for example, JP2003-153850A proposes a system that uses a rigid scope that is inserted into a body cavity.

### SUMMARY OF THE INVENTION

Here, since the above-described fluorescence of ICG is weak light, strong excitation light needs to be irradiated to a subject in order to obtain a more clear fluorescence image. Therefore, for example, a laser source which has a narrow wavelength band and less influence on the contrast between the excitation light and the fluorescence can be used as an excitation light source. However, there is a concern that high-power lasers will cause damage to eyes if viewed directly.

For example, JP2003-153850A proposes a rigid scope system in which excitation light output from a light source device is supplied to a rigid scope body through a light guide connected to the light source device. In the rigid scope system, the light guide and the rigid scope body are attachable to and detachable from each other. However, for example, it is dangerous if the light source device is accidentally left in a state where excitation light is being output when attaching the light guide to the rigid scope body or detaching the light guide from the rigid scope body after operation, because excitation light output from the connection portion of the light guide may enter the eye.

The present invention has been made in view of the above-mentioned problems and an object of the present invention is to provide an endoscope apparatus capable of preventing excitation light output from a connection portion of a light guide from entering and thereby damaging the eye when the light guide is detached.

According to an aspect of the present invention, there is provided an endoscope apparatus including: an insertion portion that is inserted into a body so as to irradiate special light to a subject observation portion inside the body and receives light output from the subject observation portion by the irradiation of the special light; and a light guiding portion that is optically connected to the insertion portion and guides the special light output from a light source to the insertion portion. An output end for the special light in the light guiding portion is equipped with a diffusing portion that diffuses the special light guided by the light guiding portion.

Further, in the endoscope apparatus of the present invention, near-infrared light may be used as the special light.

Further, a diffusing portion in which a diffusion surface diffusing the special light in the diffusing portion faces the output end of the light guiding portion may be provided.

Further, a transmissive member which has the same refractive index as that of the light guiding portion and through which the special light output from the light guiding portion is transmitted may be installed between the diffusing portion and the input end for the special light in the insertion portion. Furthermore, 'the same refractive index as that of the light guiding portion' indicates the same or almost the same refractive index and a refractive index that is almost identical can be appropriately selected by a user of the special light.

Further, a transmissive member with elasticity may be used as the transmissive member.

Further, a transmissive member formed of a resin may be used as the transmissive member.

Further, the transmissive member may be detachably installed at the output end of the light guiding portion or the input end of the insertion portion.

Further, a supply member which supplies a liquid or a gel with the same refractive index as that of the light guiding portion may be installed between the diffusing portion and the input end for the special light in the insertion portion, and the supply member may be detachably installed at the output end of the light guiding portion or the input end of the insertion portion.

Further, when the transmissive member or the supply member is provided as described above, a diffusing portion in which the diffusion surface diffusing the special light in the diffusing portion faces the input end of the insertion portion may be provided.

Further, a lens diffusing plate may be used as the diffusing portion.

Further, the insertion portion may include an optical fiber into which the special light output from the light guiding portion is input and which guides the input special light, and the input end portion for the special light in the optical fiber may be formed in a taper shape in which the core diameter increases toward the output end of the light guiding portion.

Further, the light guiding portion may also guide white light output from a white light source along with the special light, and the insertion portion may irradiate the white light guided by the light guiding portion to the subject observation portion.

According to the endoscope apparatus of the present invention, an endoscope apparatus includes: an insertion portion that is inserted into a body so as to irradiate special light to a subject observation portion inside the body and receives light output from the subject observation portion by the irradiation of the special light; and a light guiding portion that is optically connected to the insertion portion and guides the special light output from a light source to the insertion portion. An output end for the special light in the light guiding portion is equipped with a diffusing portion that diffuses the special light guided by the light guiding portion. Accordingly, it is possible to prevent excitation light output from the output end of the light guiding portion from entering and thereby damaging the eye when the light guiding portion is detached from the insertion portion.

Further, in the endoscope apparatus of the present invention, in a state where the light guiding portion is connected to the insertion portion, when a transmissive member which has the same refractive index as that of the light guiding portion and through which the special light output from the light guiding portion is transmitted is installed between the diffusing portion and the input end for the special light in the insertion portion, the special light transmission efficiency between the light guiding portion and the insertion portion may be improved.

Further, when a transmissive member with elasticity is used, since the diffusing effect may be suppressed by the diffusion surface of the diffusing portion coming into close contact with the transmissive member, the special light transmission efficiency may be further improved.

Further, in a state where the light guiding portion is connected to the insertion portion, when a supply member which supplies a liquid or a gel with the same refractive index as that of the light guiding portion is installed between the diffusing portion and the input end for the special light in the insertion portion, the special light transmission efficiency between the light guiding portion and the insertion portion may be improved. Further, when the supply member is detachably installed at the output end of the light guiding portion or the input end of the insertion portion, the supply member may be disposable.

Further, in a case where the above-described supply member is provided, when the diffusing portion in which the diffusion surface faces the input end surface of the insertion portion is provided, the diffusing effect may be suppressed in a manner such that the diffusion surface of the diffusing portion contacts the liquid or the gel, and hence the special light transmission efficiency may be improved.

Further, when the optical fiber which guides the special light is installed inside the insertion portion and the input end portion for the special light in the optical fiber has a taper shape in which the core diameter increases toward the output end of the light guiding portion, the special light condensing efficiency may be improved. Furthermore, when the core area of the output end of the optical fiber is set to be smaller than the core area of the input end of the optical fiber, the diffusion angle of the light output from the output end may be widened by the conservation rule of Etendue, and hence the special light irradiation range with respect to the subject observation portion may be widened.

### BRIEF DESCRIPTION OF THE DRAWINGS

Fig. 1 is a schematic configuration diagram illustrating a rigid scope system that uses an embodiment of an endoscope apparatus of the present invention.

Fig. 2 is a schematic configuration diagram illustrating a body cavity insertion portion.

Fig. 3 is a cross-sectional view illustrating a configuration of a light guide.

Fig. 4 is a cross-sectional view illustrating a state where the light guide and an insertion member are connected to each other.

Fig. 5 is a schematic configuration diagram illustrating an imaging unit.

Fig. 6 is a block diagram illustrating a schematic configuration of an image processing apparatus and a light source device.

Fig. 7 is a cross-sectional view illustrating another embodiment of a multi-mode optical fiber installed inside the insertion member.

Fig. 8 is a cross-sectional view illustrating an embodiment when a transmissive member is installed between an output end of the multi-mode optical fiber of the light guide and the multi-mode optical fiber of the insertion member.

Fig. 9 is a cross-sectional view illustrating an embodiment in which a supply member is installed between the light guide and a cable connection portion of the insertion member.

### DESCRIPTION OF THE PREFERRED EMBODIMENTS

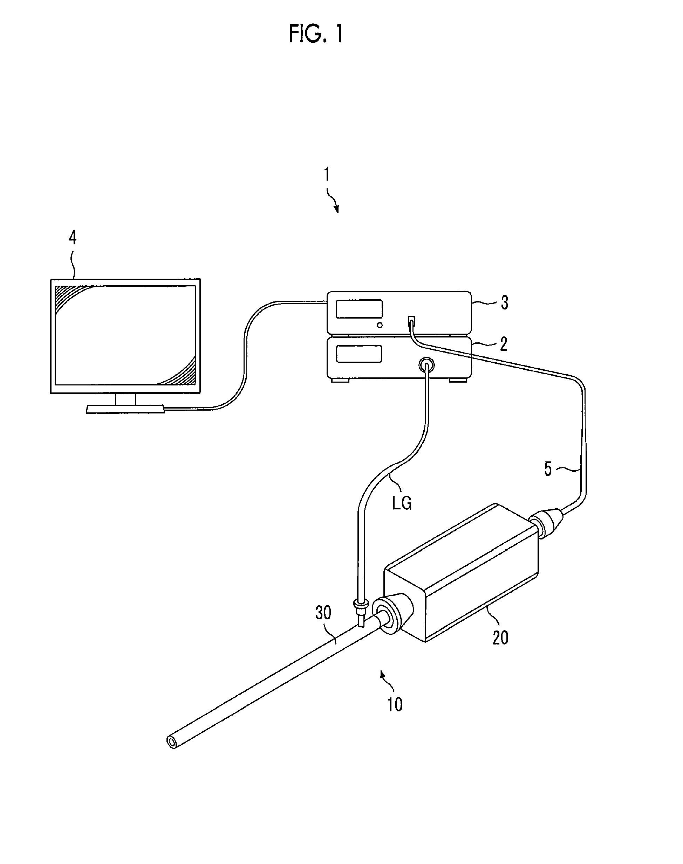

Hereinafter, a rigid scope system that uses an embodiment of an endoscope apparatus of the present invention will be described in detail by referring to the drawings. Fig. 1 is an external diagram illustrating a schematic configuration of a rigid scope system 1 of the embodiment.

As shown in Fig. 1, a rigid scope system 1 of the embodiment includes: a light source device 2 which outputs white ordinary light and excitation light (special light); a rigid scope imaging device 10 which irradiates a subject observation portion by guiding the ordinary light and the excitation light output from the light source device 2 and captures an ordinary image based on reflection light reflected from the subject observation portion by the irradiation of the ordinary light and a fluorescence image based on fluorescence output from the subject observation portion by the irradiation of the excitation light; a processor 3 which applies a predetermined process to an image signal captured by the rigid scope imaging device 10; and a monitor 4 which displays the ordinary image and the fluorescence image of the subject observation portion based on the display control signal generated in the processor 3.

As shown in Fig. 1, the rigid scope imaging device 10 includes a body cavity insertion portion 30 (an insertion portion) which is inserted into a body cavity and an imaging unit 20 which captures the ordinary image and the fluorescence image of the subject observation portion using the light guided by the body cavity insertion portion 30.

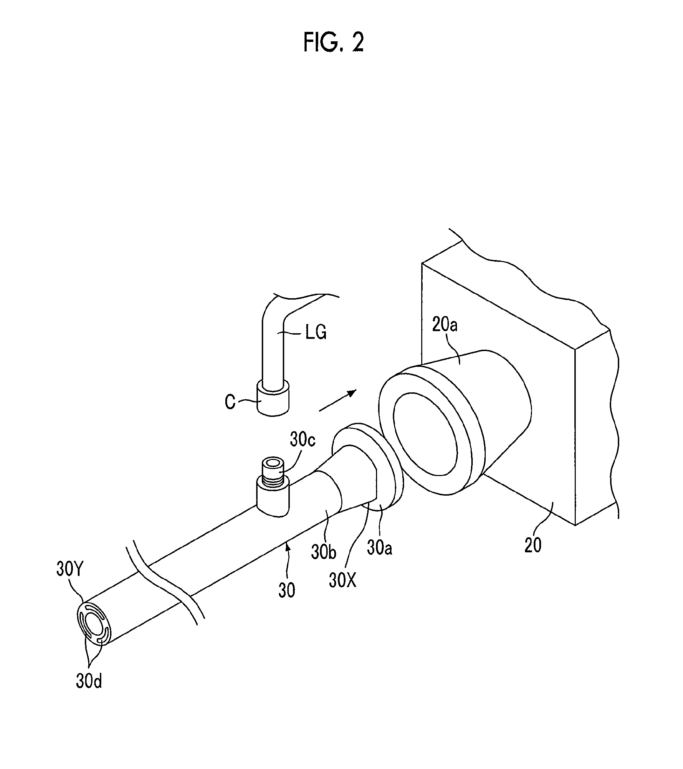

Further, in the rigid scope imaging device 10, as shown in Fig. 2, the body cavity insertion portion 30 and the imaging unit 20 are detachably connected to each other. Then, the body cavity insertion portion 30 includes a connection member 30a, an insertion member 30b, a cable connection portion 30c, and an irradiation window 30d.

The connection member 30a is installed at one end portion 30X, the imaging unit 20 side, of the body cavity insertion portion 30 (the insertion member 30b). For example, the imaging unit 20 and the body cavity insertion portion 30 are detachably connected to each other by the connection member 30a being fitted into an opening 20a formed in the imaging unit 20.

The insertion member 30b is inserted into a body cavity when image capture inside the body cavity is performed and thus is formed of a rigid material and has, for example, a cylindrical shape with a diameter of approximately 5 mm. In the inside of the insertion member 30b, a lens group which forms the image of the subject observation portion is accommodated, and a multi-mode optical fiber 31 which guides the ordinary light and the excitation light to the distal end of the insertion member 30b is bundled and extended, The details of the multi-mode optical fiber 31 will be described later.

The ordinary light and the excitation light which are input to the insertion member 30b are guided by the bundled multi-mode optical fiber 31, and are irradiated from a distal end portion 30Y toward the subject observation portion. Then, the ordinary image and the fluorescence image which are output from the subject observation portion by the irradiation of the ordinary light and the excitation light are input from the distal end portion 30Y, and are output from an end portion 30X, the imaging unit 20 side, through the above-described lens group.

The cable connection portion 30c is equipped on the side surface of the insertion member 30b ,and the light guide LG (the light guiding portion) is mechanically connected to the cable connection portion 30c through a connector C. Accordingly, the light source device 2 and the insertion member 30b are optically connected to each other through the light guide LG

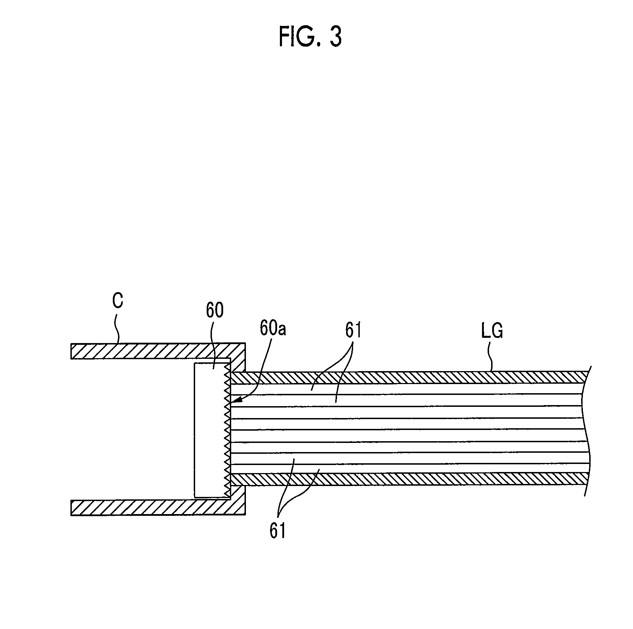

In the inside of the light guide LG, as shown in Fig. 3, the ordinary light and the excitation light are input from one end thereof, and a multi-mode optical fiber 61 which guides the input ordinary light and the input excitation light is bundled and extended.

Then, the output end portion for the ordinary light and the excitation light in the multi-mode optical fiber 61 is equipped with the connector C which is fitted and connected to the cable connection portion 30c of the insertion member 30b, and the inside of the connector C is equipped with a diffusing portion 60 which is optically connected to the output end surface of the multi-mode optical fiber 61. As the diffusing portion 60, for example, a lens diffusing plate (LSD: Light shaping Diffusers), a diffusing film, a diffusing sheet, a diffusing filter, and the like may be used. That is, the diffusing portion may be formed as any type through which input light is transmitted in a diffusing manner.

Then, for example, when a lens diffusing plate is used as the diffusing portion 60, as shown in Fig. 3, it is desirable to install the lens diffusing plate so that the diffusion surface 60a having a surface-relief-hologram pattern formed thereon becomes the input surface for the ordinary light and the excitation light, that is, the diffusion surface 60a becomes the light output end surface of the multi-mode optical fiber 61. Since the diffusion surface 60a is disposed in the above-described direction, it is possible to prevent a problem in which dirt or water adheres to the diffusion surface so that the diffusing function degrades.

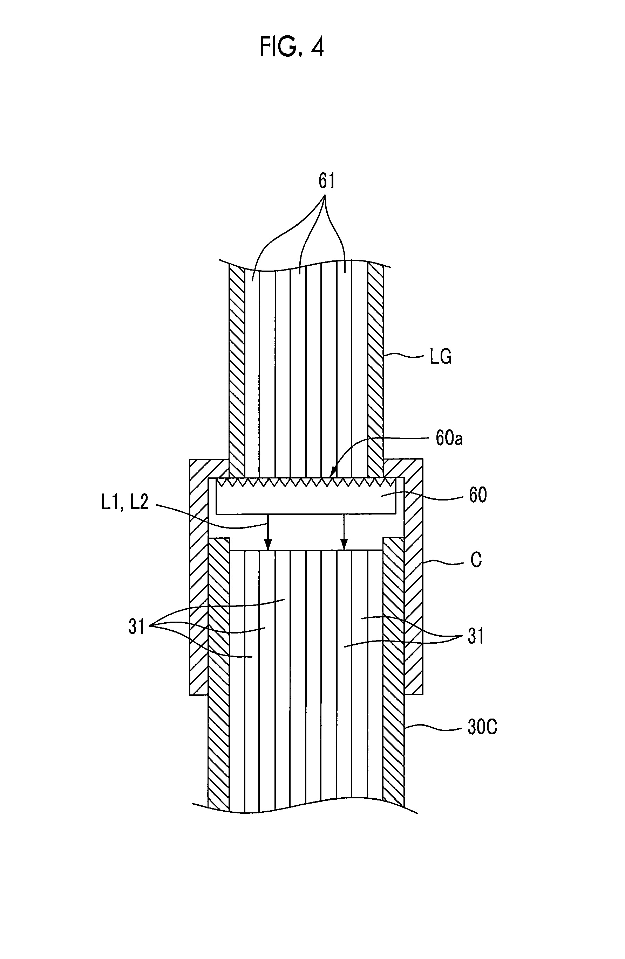

Fig. 4 illustrates a state where the connector C of the light guide LG is connected to the cable connection portion 30c of the insertion member 30b. As shown in Fig. 4, in the embodiment, a gap is formed between the light output surface of the diffusing portion 60 and the light input surface of the multi-mode optical fiber 31 installed inside the insertion member 30b in a state where the light guide LG is connected to the insertion member 30b. Accordingly, it is possible to prevent the diffusing portion 60 and the multi-mode optical fiber 31 from being damaged by a collision therebetween.

Then, the ordinary light L1 and the excitation light L2 which are guided by the multi-mode optical fiber 61 inside the light guide LG are input to the diffusing portion 60, diffused in the diffusion surface 60a of the diffusing portion 60, and after being output are input from the input surface of the multi-mode optical fiber 31 installed inside the insertion member 30b.

Then, the distal end portion 30Y of the insertion member 30b is equipped with an irradiation window 30d as shown in Fig. 2. The irradiation window 30d irradiates the ordinary light and the excitation light which are guided by the multi-mode optical fiber 31 extending inside the insertion member 30b to the subject observation portion.

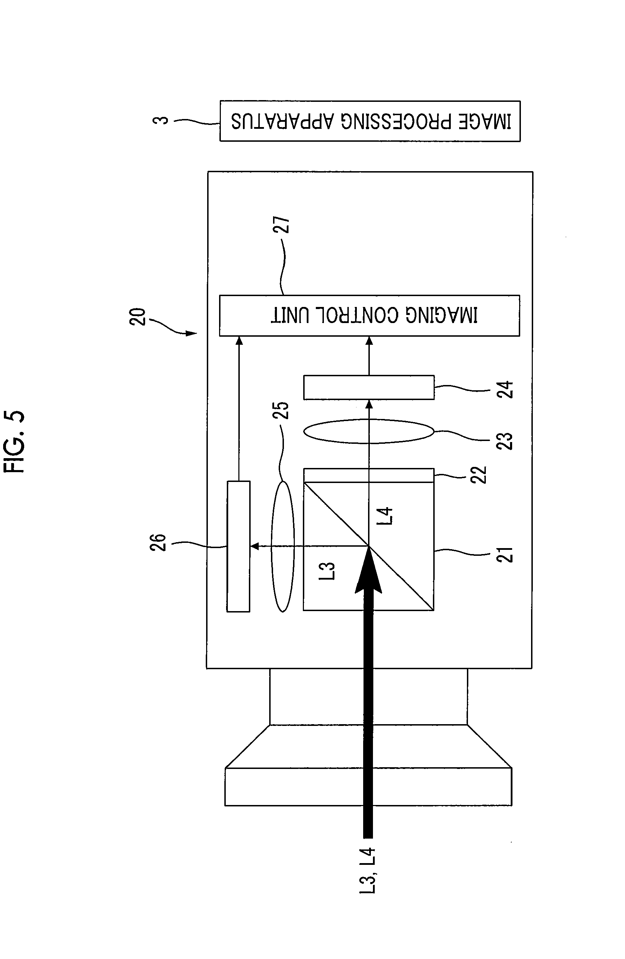

Fig. 5 is a diagram illustrating a schematic configuration of the imaging unit 20. The imaging unit 20 includes a first imaging system which generates a fluorescence image signal of the subject observation portion by capturing the fluorescence image of the subject observation portion formed by the lens group inside the body cavity insertion portion 30 and a second imaging system which generates an ordinary image signal by capturing the ordinary image of the subject observation portion formed by the lens group inside the body cavity insertion portion 30. These imaging systems are divided into two optical axes perpendicular to each other by dichroic prisms 21 which have spectral characteristics which reflect the ordinary image and cause the fluorescence image to be transmitted therethrough.

The first imaging system includes an excitation light cut-off filter 22, a first imaging optical system 23 and a high-sensitive imaging element 24. The excitation light cut-off filter 22 cuts light at a wavelength lower than that of the excitation light reflected from the subject observation portion and transmitted through the dichroic prism 21, and causes fluorescence wavelength band illumination light to be described later to be transmitted therethrough. The first imaging optical system 23 forms a fluorescence image L4 output from the body cavity insertion portion 30 and transmitted through the dichroic prism 21 and the excitation light cut-off filter 22. The high-sensitive imaging element 24 captures the fluorescence image L4 formed by the first imaging optical system 23.

The high-sensitive imaging element 24 has a high sensitivity for detecting light of the wavelength band of the fluorescence image L4 and converts it into a fluorescence image signal and outputs this signal. As the high-sensitive imaging element 24, for example, a monochrome imaging element may be used.

The second imaging system includes a second imaging optical system 25 and an imaging element 26. The second imaging optical system 25 forms ordinary image L3 output from the body cavity insertion portion 30 and reflected from the dichroic prism 21. The imaging element 26 captures an ordinary image L3 formed by the second imaging optical system 25.

The imaging element 26 detects light of the wavelength band of the ordinary image, converts it into an ordinary image signal and outputs this signal. In the imaging surfaces of the imaging element 26, color filters of three primary colors, red (R), green (G), and blue (B), or cyan (C), magenta (M), and yellow (Y) are arranged according to a Bayer arrangement or a honeycomb arrangement.

Further, the imaging unit 20 includes an imaging control unit 27. The imaging control unit 27 performs a CDS/AGC (correlated double sampling/automatic gain control) process or an A/D conversion process on the fluorescence image signal output from the high-sensitive imaging element 24 and the ordinary image signal output from the imaging element 26, and outputs the result to the processor 3 through the cable 5 (see Fig. 1).

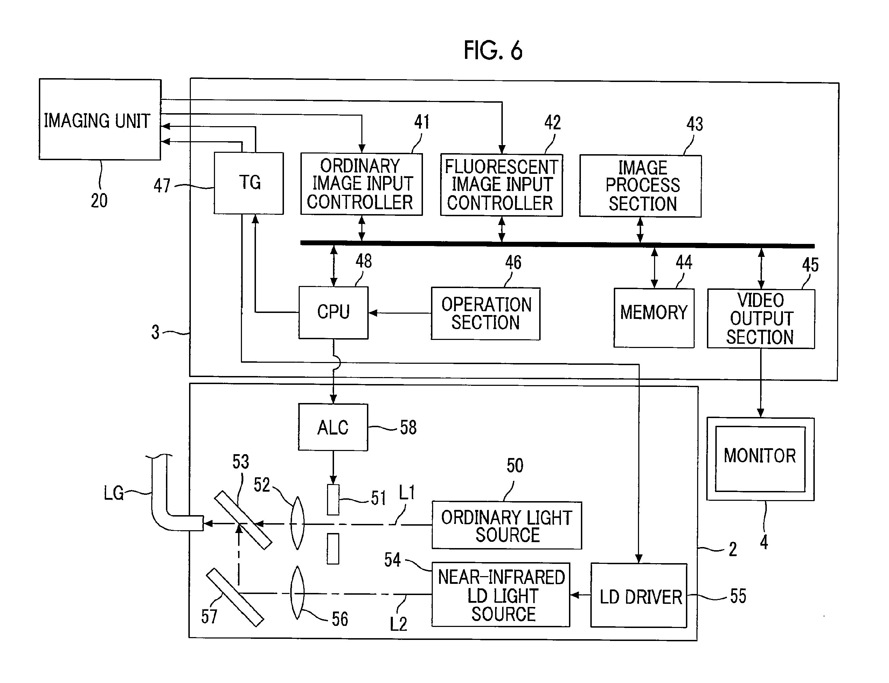

As shown in Fig. 6, the processor 3 includes an ordinary image input controller 41, a fluorescence image input controller 42, an image process section 43, a memory 44, a video output section 45, an operation section 46, a TG (a timing generator) 47, and a CPU 48.

The ordinary image input controller 41 and the fluorescence image input controller 42 include a line buffer with a predetermined capacity, and the ordinary image input controller 41 temporarily stores the ordinary image signal for each frame output from the imaging control unit 27 of the imaging unit 20, and the fluorescence image input controller 42 temporarily stores the fluorescence image signal. Then, the ordinary image signal stored in the ordinary image input controller 41 and the fluorescence image signal stored in the fluorescence image input controller 42 are stored in the memory 44 through a bus.

The image process section 43 receives the ordinary image signal and the fluorescence image signal for each frame read out from the memory 44, performs a predetermined image process on the image signals, and outputs the result to the bus.

The video output section 45 receives the ordinary image signal and the fluorescence image signal output from the image process section 43 through the bus, performs a predetermined process thereon so as to generate a display control signal, and outputs the display control signal to the monitor 4.

The operation section 46 receives an input from an operator, such as various operation instructions or control parameters. Further, the TG 47 outputs driving pulses for driving the high-sensitive imaging element 24 of the imaging unit 20, the imaging element 26, and the LD driver 55 of the light source device 2 to be described later. Further, the CPU 48 controls the entire apparatus.

The light source device 2 includes: an ordinary light source 50 which outputs the ordinary light (white light) L1 and is formed by a wide band wavelength of about 400 to 700 nm; a condenser lens 52 which condenses the ordinary light L1 output from the ordinary light source 50; and a dichroic mirror 53 through which the ordinary light L1 condensed by the condenser lens 52 is transmitted and which reflects the excitation light L2 to be described later so that the ordinary light L1 and the excitation light L2 are input to the input end of the multi-mode optical fiber 61 of the light guide LG Furthermore, as the ordinary light source 50, for example, a xenon lamp is used. Further, an aperture 51 is installed between the ordinary light source 50 and the condenser lens 52, and the amount of aperture value is controlled based on the control signal from the ALC (Automatic light control) 58.

Further, the light source device 2 includes: a near-infrared LD light source 54 which outputs near-infrared light of 750 to 790 nm as the excitation light L2; the LD driver 55 which drives the near-infrared LD light source 54; a condenser lens 56 which condenses the excitation light L2 output from the near-infrared LD light source 54; and a mirror 57 which reflects the excitation light L2 condensed by the condenser lens 56 toward the dichroic mirror 53.

Furthermore, as the excitation light L2, a wavelength which is narrower than that of the ordinary light formed by the wide band wavelength is used. Then, the wavelength of the excitation light L2 is not limited to the light of the above-described wavelength band, and may be appropriately determined by the type of fluorescent pigment or the type of self fluorescent body tissue.

Next, the operation of the rigid scope system of the embodiment will be described.

First, the connector C of the light guide LG connected to the light source device 2 is connected to the cable connection portion 30c of the insertion member 30b of the body cavity insertion portion 30, and the cable 5 connected to the processor 3 is connected to the imaging unit 20.

At this time, in general, since the power of the light source device 2 is turned off, no light is output from the connector C of the light guide LG However, for example, in a state where the power of the light source device 2 is mistakenly in the on state, the excitation light L2 output from the light source device 2 may be guided by the light guide LG so as to output to the outside from the connector C.

However, even in such a state, as described above, since the excitation light L2 is diffused by the diffusing portion 60 installed at the output end of the multi-mode optical fiber 61, the energy density of the excitation light L2 may be decreased, and safety may be ensured even when the excitation light L2 enters the eye.

Then, in general, the power of the light source device 2 is turned on after the light guide LG and the cable 5 are respectively connected to the body cavity insertion portion 30 and the imaging unit 20, the body cavity insertion portion 30 is inserted into the body cavity by the operator, and the distal end of the body cavity insertion portion 30 is installed near the subject observation portion.

Then, the ordinary light L1 output from the ordinary light source 50 of the light source device 2 and the excitation light L2 output from the near-infrared LD light source 54 are guided by the multi-mode optical fiber 61 of the light guide LG, and after being output from the output end of the multi-mode optical fiber 61, are input to the diffusing portion 60. The ordinary light L1 and the excitation light L2 which are input to the diffusing portion 60 are diffused in the diffusion surface 60a of the diffusing portion 60, output and then input to the input end of the multi-mode optical fiber 31 inside the insertion member 30b.

The ordinary light L1 and the excitation light L2 which are guided by the multi-mode optical fiber 31 inside the insertion member 30b are irradiated toward the subject observation point from the irradiation window 30d installed in the distal end portion 30Y of the insertion member 30b.

Subsequently, the ordinary image based on the reflection light reflected from the subject observation portion by the irradiation of the ordinary light L1 is captured, and the fluorescence image based on the fluorescence output from the subject observation portion by the irradiation of the excitation light L2 is captured. Furthermore, ICG is input to the subject observation portion in advance, and the fluorescence output from the ICG is captured.

Specifically, at the time of capturing the ordinary image, the ordinary image L3 based on the reflection light reflected from the subject observation portion by the irradiation of the ordinary light L1 is input from the distal end portion 30Y of the insertion member 30b, is guided by the lens group inside the insertion member 30b, and is output toward the imaging unit 20.

The ordinary image L3 which is input to the imaging unit 20 is reflected in the perpendicular direction by the dichroic prism 21 toward the imaging element 26, is formed on the imaging surface of the imaging element 26 by the second imaging optical system 25, and is sequentially captured at a predetermined frame rate by the imaging element 26.

The ordinary image signal which is sequentially output from the imaging element 26 is processed by a CDS/AGC (correlated double sampling/automatic gain control) process or an A/D conversion process in the imaging control unit 27, and is sequentially output to the processor 3 through the cable 5.

Then, the ordinary image signal which is input to the processor 3 is temporarily stored in the ordinary image input controller 41, and is stored in the memory 44. Then, the ordinary image signal for each frame read out from the memory 44 is processed by a grayscale correction process and a sharpness correction process in the image process section 43, and is sequentially output to the video output section 45.

Then, the video output section 45 performs a predetermined process on the ordinary image signal which is input, generates a display control signal, and sequentially outputs the display control signal for each frame to the monitor 4. Then, the monitor 4 displays the ordinary image based on the input display control signal.

On the other hand, at the time of capturing the fluorescence image, the fluorescence image L4 based on the fluorescence output from the subject observation portion by the irradiation of the excitation light is input from the distal end portion 30Y of the insertion member 30b, is guided by the lens group inside the insertion member 30b, and is output toward the imaging unit 20.

The fluorescence image L4 which is input to the imaging unit 20 passes through the dichroic prism 21 and the excitation light cut-off filter 22, is formed on the imaging surface of the high-sensitive imaging element 24 by the first imaging optical system 23, and is captured at a predetermined frame rate by the high-sensitive imaging element 24.

The fluorescence image signal which is sequentially output from the high-sensitive imaging element 24 processed by a CDS/AGC (correlated double sampling/automatic gain control) process or an A/D conversion process in the imaging control unit 27, and is sequentially output to the processor 3 through the cable 5.

Then, the fluorescence image signal which is input to the processor 3 is temporarily stored in the fluorescence image input controller 42, and is stored in the memory 44. Then, the fluorescence image signal for each frame read out from the memory 44 undergoes a predetermined image process in the image process section 43, and is sequentially output to the video output section 45.

Then, the video output section 45 performs a predetermined process on the fluorescence image signal which is input, generates a display control signal, fluorescence image and sequentially outputs the display control signal for each frame to the monitor 4. Then, the monitor 4 displays the fluorescence image based on the input display control signal.

Then, after the ordinary image and the fluorescence image have been captured, in general, the power of the light source device 2 is turned off and then the light guide LG is detached from the cable connection portion 30c of the insertion member 30b, so that no light is output from the detached connector C of the light guide LG However, for example, the light guide LG may be detached from the cable connection portion 30c of the insertion member 30b in a state where the power of the light source device 2 is accidentally turned on.

However, as described above, since the excitation light L2 is diffused by the diffusing portion 60 installed at the output end of the multi-mode optical fiber 61, the energy density may be decreased, and the safety may be ensured even when the excitation light L2 enters the eye. Furthermore, the same applies to the case where the light guide LG is accidentally detached from the cable connection portion 30c of the insertion member 30b when capturing the ordinary image and the fluorescence image.

Further, in the above-described embodiment, the diffusing portion 60 is installed at the output end of the light guide LG in order to ensure safety with respect to the excitation light. However, when the diffusing portion 60 is installed in this way, the ordinary light L1 and the excitation light L2 are diffused and input to the multi-mode optical fiber 31 of the insertion member 30b, which is not desirable from the viewpoint of light transmission efficiency.

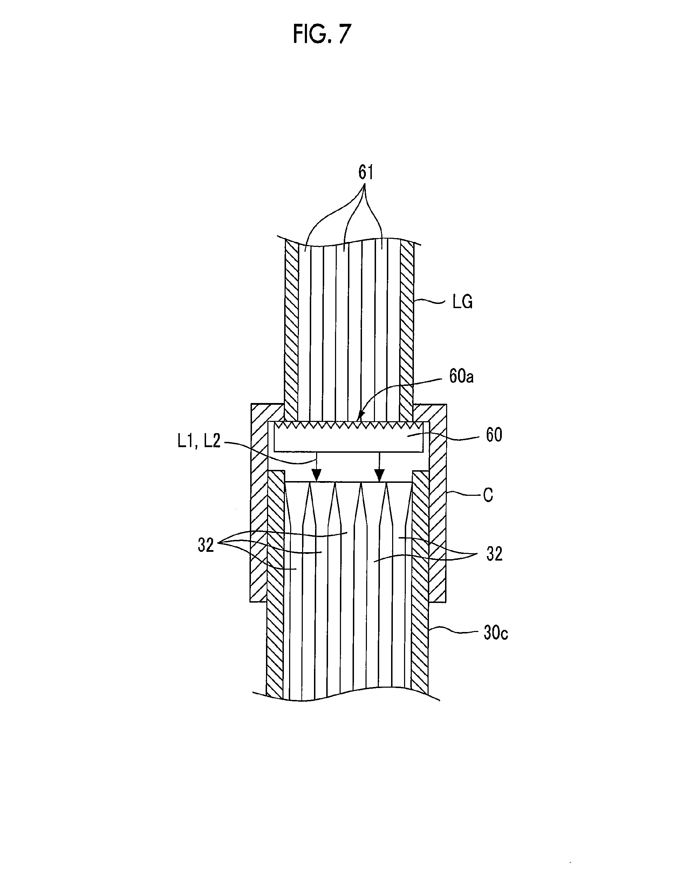

Therefore, for example, as shown in Fig. 7, the inside of the insertion member 30b may be equipped with a multi-mode optical fiber 32 having a taper portion in which the core diameter gradually increases toward the input ends of the ordinary light L1 and the excitation light L2. Accordingly, the condensing efficiency of the ordinary light L1 and the excitation light L2 output from the diffusing portion 60 may be improved, and the transmission efficiency may be improved. Further, in the case where the input end of the multi-mode optical fiber 32 has the taper portion in this way, when the core area of the output end of the multi-mode optical fiber 32 is smaller than the core area of the input end, since the diffusion angle of the light output from the output end may be widened by the conservation rulel of Etendue, it is more desirable in that the irradiation ranges of the ordinary light L1 and the excitation light L2 with respect to the subject observation portion may be widened.

Furthermore, when the multi-mode optical fiber 32 shown in Fig. 7 is used, the diffusion surface 60a of the diffusing portion 60 may be disposed at the input surface side of the multi-mode optical fiber 32.

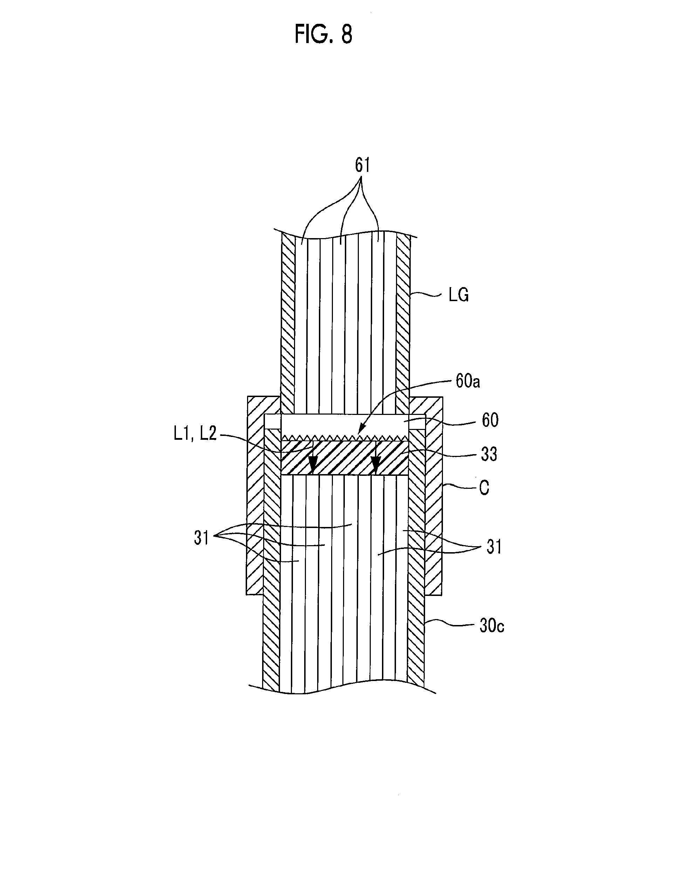

Further, methods for improving the transmission efficiency between the light guide LG and the multi-mode optical fiber 31 of the insertion member 30b are not limited thereto. For example, as shown in Fig. 8, a transmissive member 33 which has the same refractive index as that of the multi-mode optical fiber 31 of the light guide LG and through which the ordinary light L1 and the excitation light L2 output from the light guide LG are transmitted may be installed inside the cable connection portion 30c of the insertion member 30b. Since the transmissive member 33 is installed, when the light guide LG and the insertion member 30b are connected to each other, the diffusing portion 60 may be optically connected to the multi-mode optical fiber 31 of the insertion member 30b, and the transmission efficiency of the ordinary light L1 and the excitation light L2 may be improved.

Further, as the transmissive member 33, it is desirable to use a material having elasticity, and for example, it is desirable to use a member formed from resin. As the transmissive member 33, for example, a silicon material for LED (SLJ9101, SLJ9102, SLJ9105, and SLJ9106 (Asahi Kasei Corporation.)) or highly transparent urethane rubber may be used.

When the transmissive member 33 with elasticity is used in this way, the adhesion between the diffusing portion 60 and the transmissive member 33 and the adhesion between the transmissive member 33 and the input surface of the multi-mode optical fiber 31 may be improved.

In particular, as shown in Fig. 8, when the diffusion surface 60a of the diffusing portion 60 is disposed to face the input surface side of the multi-mode optical fiber 31, the adhesion between the diffusion surface 60a of the diffusing portion 60 and the transmissive member 33 is improved so that the diffusing effect in the diffusion surface 60a may be suppressed. Accordingly, the transmission efficiency of the ordinary light L1 and the excitation light L2 may be further improved.

However, the direction of the diffusion surface 60a is not limited thereto, and as in the above-described embodiment, the direction may face the light output end surface side of the multi-mode optical fiber 61.

Further, it is desirable that the above-described transmissive member 33 is detachably installed in the cable connection portion 30c of the insertion member 30b. With such a configuration, for example, even when dirt or the like adheres to the transmissive member 33, the transmissive member 33 may be detached so as to remove dirt therefrom and be reattached thereto. As a configuration in which the transmissive member 33 is detachably installed, for example, the transmissive member 33 may be freely extracted by a hand without being fixed to the cable connection portion 30c, or the transmissive member 33 may be provided in a member which is attachable to and detachable from the cable connection portion 30c.

Further, instead of the transmissive member 33 being installed in the cable connection portion 30c of the insertion member 30b, it may be installed inside the connector C of the light guide LG so as to contact the diffusion surface 60a of the diffusing portion 60.

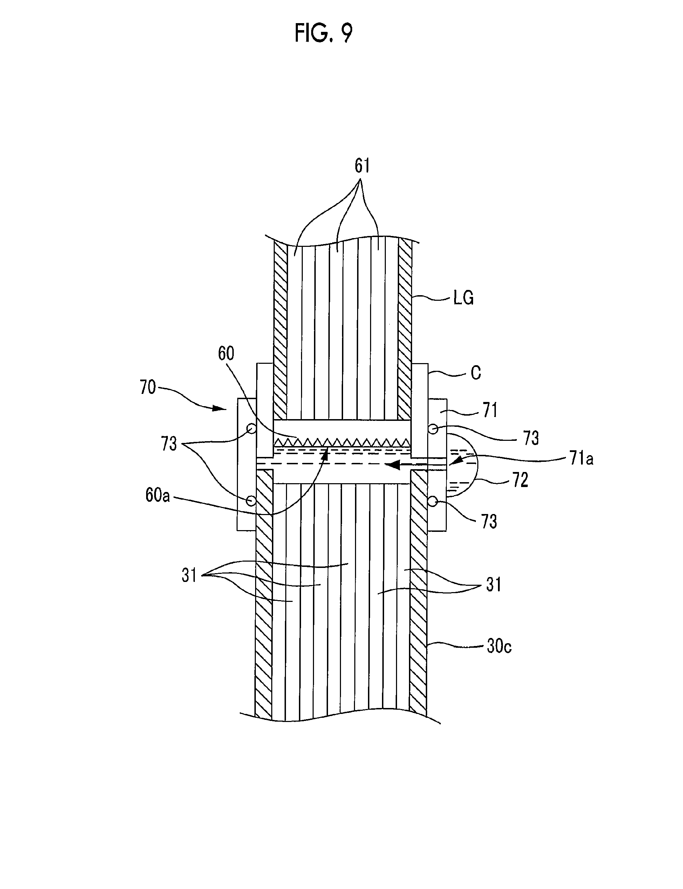

Further, as shown in Fig. 9, a supply member 70 which supplies a liquid or a gel and has the same refractive index as that of the multi-mode optical fiber 61 may be installed between the light guide LG and the cable connection portion 30c of the insertion member 30b.

The supply member 70 includes a cylindrical connection portion 71 which connects the connector C of the light guide LG and the cable connection portion 30c of the insertion member 30b to each other by fitting together and an accumulation portion 72 which is installed in the connection portion 71 and accumulates the above-described liquid or gel. Then, the connection portion 71 is detachably installed to the connector C of the light guide LG and the cable connection portion 30c of the insertion member 30b, so that the supply member 70 is disposable. Further, the accumulation portion 72 is formed of a material such as a resin which is deformable by applying pressure with the hand, and the liquid or the gel charged therein has the same refractive index as that of the multi-mode optical fiber 61. An example of such a liquid includes matching oil, a matching gel, or the like. For example, a refractive liquid which is manufactured by Cargill Japan Limited and described in the website of http://www.cargille.com/refractivestandards.shtml may be used.

Then, the connector C of the light guide LG and the cable connection portion 30c of the insertion member 30b are connected to the supply member 70 by fitting together, and a liquid or the like inside the accumulation portion 72 is discharged through a hole 71a formed in the connection portion 71 when the accumulation portion 72 is deformed by applying pressure with the hand..

The ejected liquid or the like is supplied between the diffusing portion 60 of the light guide LG and the input surface of the multi-mode optical fiber 31 of the insertion member 30b, so that the diffusing portion 60 and the multi-mode optical fiber 31 may be optically connected to each other and the transmission efficiency may be improved. Furthermore, the connection portion 71 is equipped with a water tightness ring 73 which prevents the leakage of the liquid supplied as described above.

Furthermore, when the above-described supply member 70 is used, the diffusing portion 60 may be provided as shown in Fig. 9 so that the diffusion surface 60a faces the input end side of the multi-mode optical fiber 31. With such a configuration, since the diffusing effect in the diffusion surface 60a may be suppressed in a manner such that the diffusion surface 60a of the diffusing portion 60 contacts a liquid or the like supplied from the supply member 70, the transmission efficiency of the ordinary light L1 and the excitation light L2 may be improved.

However, the direction of the diffusion surface 60a is not limited thereto, and as in the above-described embodiment, the direction may face the light output end side of the multi-mode optical fiber 61. In this way, when the diffusing portion 60 is installed, it is possible to prevent a reduction in the diffusing effect in a manner such that a liquid or the like which is supplied from the supply member 70 adheres to the diffusion surface 60a.

Furthermore, in the above-described embodiment, the fluorescence image is captured by the first imaging system, but the present invention is not limited thereto. That is, an image based on the light absorbing characteristic of the subject observation portion by the irradiation of the special light to the subject observation portion may be captured.

Further, in the above-described embodiment, a case has been described in which the endoscope apparatus of the present invention is applied to the rigid scope system, but the present invention is not limited thereto. For example, the present invention may be applied to a soft endoscope system.

## Claims

1. An endoscope apparatus comprising:
an insertion portion that is inserted into a body so as to irradiate special light to a subject observation portion inside the body and receives light output from the subject observation portion by the irradiation of the special light; and
a light guiding portion that is optically connected to the insertion portion and guides the special light output from a light source to the insertion portion,
wherein an output end for the special light in the light guiding portion is equipped with a diffusing portion that diffuses the special light guided by the light guiding portion.

2. The endoscope apparatus according to claim 1,
wherein the special light is near-infrared light.

3. The endoscope apparatus according to claim 1 or 2,
wherein a diffusion surface diffusing the special light in the diffusing portion faces the output end side of the light guiding portion.

4. The endoscope apparatus according to claim 1 or 2,
wherein a transmissive member which has the same refractive index as that of the light guiding portion and through which the special light output from the light guiding portion is transmitted is installed between the diffusing portion and the input end for the special light in the insertion portion.

5. The endoscope apparatus according to claim 4,
wherein the transmissive member has elasticity.

6. The endoscope apparatus according to claim 4 or 5,
wherein the transmissive member is formed of a resin.

7. The endoscope apparatus according to any one of claims 4 to 6,
wherein the transmissive member is detachably installed in the output end of the light guiding portion or the input end of the insertion portion.

8. The endoscope apparatus according to claim 1 or 2,
wherein a supply member which supplies a liquid or a gel with the same refractive index as that of the light guiding portion is installed between the diffusing portion and the input end for the special light in the insertion portion, and
wherein the supply member is detachably installed in the output end of the light guiding portion or the input end of the insertion portion.

9. The endoscope apparatus according to any one of claims 4 to 8,
wherein the diffusion surface diffusing the special light in the diffusing portion faces the input end side of the insertion portion.

10. The endoscope apparatus according to any one of claims 1 to 9,
wherein the diffusing portion is a lens diffusing plate.

11. The endoscope apparatus according to any one of claims 1 to 10,
wherein the insertion portion includes an optical fiber into which the special light output from the light guiding portion is input and which guides the input special light, and
wherein an input end portion for the special light in the optical fiber has a taper shape in which a core diameter gradually increases toward the output end side of the light guiding portion.

12. The endoscope apparatus according to any one of claims 1 to 11,
wherein the light guiding portion also guides white light output from a white light source along with the special light, and
wherein the insertion portion irradiates the white light guided by the light guiding portion to the subject observation portion.
